# EUROPÄISCHE PATENTSCHRIFT

(11) **EP 1 551 864 B1**
(45) Veröffentlichungstag und Bekanntmachung des Hinweises auf die Patenterteilung: **09.11.2011**
(21) Anmeldenummer: 02781041.5
(22) Anmeldetag: 06.12.2002
(51) Int. Cl.: C12Q 1/56, C07K 5/06, C07K 5/08

(54) **Substrate zur Bestimmung von TAFI(a)**
Substrates for detection of TAFI(a)
Substrats pour la détection de TAFI(a)

(30) Priorität: 04.10.2002 WO PCT/CH02/00553
(43) Veröffentlichungstag der Anmeldung: 13.07.2005
(73) Patentinhaber: DSM IP Assets B.V., 6411 TE Heerlen (NL)
(72) Erfinder: ZIEGLER, Hugo, CH-4108 Witterswil (CH); PRASA, Dagmar, 99094 Erfurt (DE); STÜRZEBECHER, Jörg, 99094 Erfurt (DE); WIKSTROEM, Peter, CH-5073 Gipf-Oberfrick (CH)
(74) Vertreter: Braun, André jr.
(86) Internationale Anmeldenummer: PCT/CH2002/000670
(87) Internationale Veröffentlichungsnummer: WO 2004/031216

(56) Entgegenhaltungen:
- HONG ET AL: "Development of substrate for carboxypeptidase-B by employing thiaarginine peptides" BULLETIN OF THE KOREAN CHEMICAL SOCIETY, Bd. 19, Nr. 2, 1998, Seiten 189-193, XP008018429 in der Anmeldung erwähnt
- BOFFA ET AL: "Plasma and recombinant thrombin-activable fibrinolysis inhibitor (TAFI) and activated TAFI compared with respect to glycosylation, thrombin/thrombomodulin-dependent activation, thermal stability, and enzymatic properties" JOURNAL OF BIOLOGICAL CHEMISTRY, Bd. 273, Nr. 4, 23. Januar 1998 (1998-01-23), Seiten 2127-2135, XP002183690 ISSN: 0021-9258

## Beschreibung

TAFI (thrombin-activatable fibrinolysis inhibitor) ist ein der Carboxypeptidase B ähnliches Proenzym von 55 kDa, welches durch Proteolyse am Arg-92 zum Enzym TAFIa (35 kDa) aktiviert wird. Im Unterschied zur Caboxypeptidase B, welche als Proenzym von 46 kDa durch Proteolyse am Arg-95 aktiviert wird und im Magen und Verdauungstrakt wirkt, ist TAFIa ein Leberenzym, das seine Wirksamkeit im Blut entfaltet.

TAFIa wird durch Thrombin und wahrscheinlich auch Plasmin aus TAFI gebildet und ist ein wichtiger Inhibitor der Fibrinolyse. Die Aktivierung von TAFI wird durch die Bindung von Thrombin an Thrombomodulin ungefähr 1250-fach verstärkt. Die inhibierende Wirkung von TAFIa beruht auf der Fähigkeit, Carboxy-terminale Arginine und Lysine abzuspalten, wobei die Spezifität gegenüber Arginin grösser ist.

TAFIa selbst ist gegenüber Proteolysen sehr empfindlich und bereits bei 37°C instabil.

Fibrinolyse beginnt, wenn Plasminogen durch tPA in Plasmin umgewandelt wird. Plasmin baut dann das Fibringerinnsel zu löslichen Fibrin-Produkten ab, die ihrerseits zu einer zusätzlichen Stimulierung der Plasminbildung beitragen. TAFIa spaltet die Carboxy-terminalen Lysine dieser Fibrin Produkte ab, was zu einer Verhinderung der Bildung des tPA/Plasminogen/Fibrin-Komplexes führt, womit die Fibrinolyse gehemmt wird.

Die Messung der TAFla-Konzentration im Plasma gibt einen wichtigen Hinweis zum Blutungs- resp.Thromboserisiko von Patienten.

Eine Bestimmung von Carboxypeptidasen ist möglich mit immunologischen Tests, wie z.B. ELISA, welche aber nicht die Aktivität von TAFI erfassen. Funktionelle Tests sind beschrieben, worin TAFIa auf synthetische Substrate des Typs R-Arg-COOH oder R-Lys-COOH einwirkt und dabei 'R' (z.B. Hippuryl) freisetzt, welches mittels HPLC oder spektrophotometrisch im nahem UV-Bereich gemessen werden kann.

Es gibt bisher erst einen chromogenen Assay für die Messung von TAFI und TAFIa im Plasma auf Mikrotiterplatten. Die Farbstoffbildung ist jedoch zu kompliziert (mehrstufig) und verläuft unbefriedigend. Da die Absorptionsmessung bei 490 nm vorgenommen wird, eignet er sich - wie auch die vorgängig beschriebenen Methoden - nicht für die Messung auf gängigen Automaten, in denen die Extinktionsänderungen bei 405 nm erfolgen.

Die Thiaarginin-Derivate der folgenden Formeln A und B 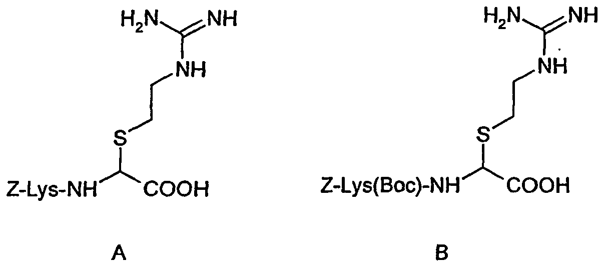 sind als Substrate für Carboxypeptidase B beschrieben worden (Bull. Korean. Chem. Soc. 1998, 19(2), 189-193). Diese Verbindungen zeigen bemerkenswerte Spaltungsraten durch Carboxypeptidase B (CPB), andererseits werden sie durch TAFIa kaum gespalten. Der Nachweis von CPB mittels Verbindung A erfolgt gemäss dem nachfolgenden Schema 1.

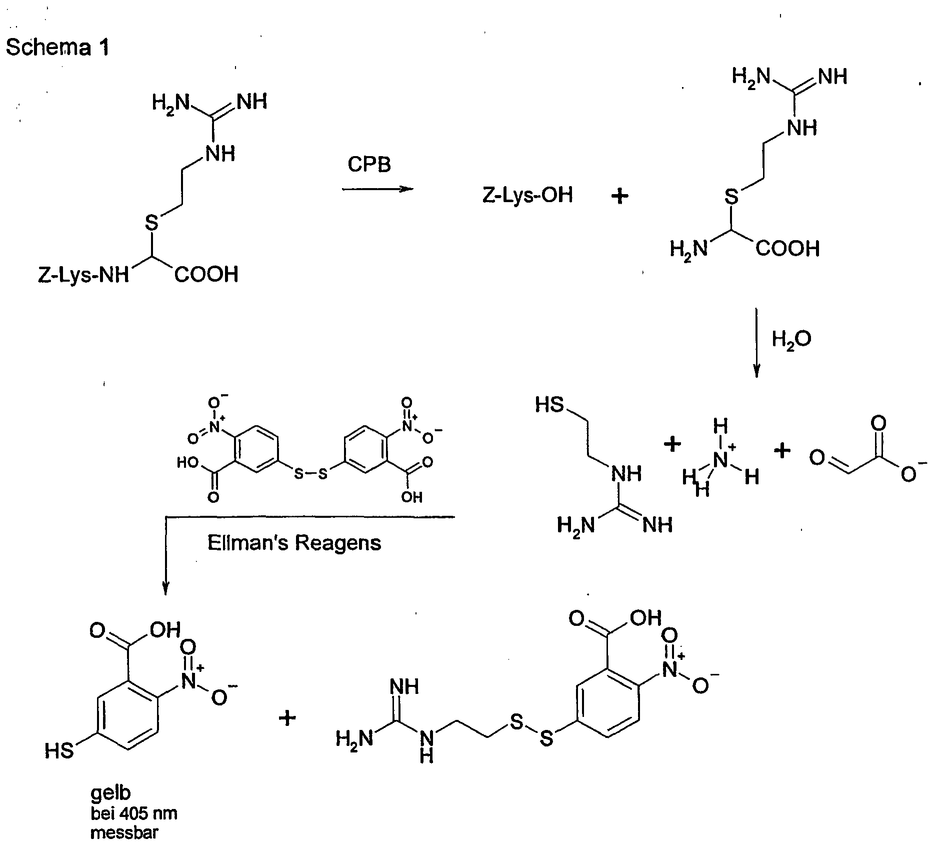

Überraschenderweise lässt sich diese Selektivität bereits durch kleine Strukturvariationen zu Gunsten von TAFIa umkehren.

Die vorliegende Erfindung betrifft die Verwendung von TAFIa-Substrate der allgemeinen Formel I 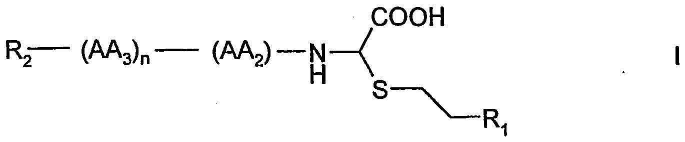 worin
- R₁: eine Gruppe CH₂NH₂ oder NHC(NH)NH₂ bedeutet,
- AA₂: jeweils unsubstituiertes oder substituiertes Lysin, Ornithin, Arginin oder Histidin bedeutet, wobei die Substituenten übliche Schutzgruppen sind,
- AA₃: eine natürliche Aminosäure bedeutet, bei der eine allenfalls vorhandene schützbare Gruppe in der Seitenkette durch eine übliche Schutzgruppe substituiert sein kann,
- n: 0 oder 1 ist, und
- R₂: eine Gruppe Bz, Bzl, Ac, Boc, Z, Suc, MeoSuc oder Tos bedeutet,
mit der Massgabe, dass nicht gleichzeitig n 0 ist und R₁ NHC(NH)NH₂, R₂ Z und (AA₂) unsubstituiertes oder durch Boc substituiertes Lysin bedeuten,
als Racemate oder als enantiomerenreine Isomere,
und deren Salze mit Mineralsäuren oder organischen Säuren.

R₁ bedeutet vorzugsweise NHC(NH)NH₂.

Bevorzugte Bedeutungsmöglichkeiten für AA₂ sind z.B. Lys(ε-Z), Lys(ε-Boc), Lys(ε-Ac), Lys(ε-Bz), Lys(ε-Bzl), Lys(ε-Tos), Orn(δ-Z), Orn(δ-Boc), Orn(δ-2-chlor-Z), Orn(δ-Dnp), Orn(δ-Z), Orn(δ-Aloc), Arg(ω-Pbf), Arg(δ,ω-Boc)₂, Arg(δ,ω-Z)₂, Arg(ω-Tos), His(N^{im}-Boc), His(N^{im}-Ac), His(N^{im}-Bz),His(N^{im}-Bzl) oder His(N^{im}-Tos), wobei Lys(ε-Z) besonders bevorzugt ist.

Als Schutzgruppen in der Aminosäure AA₃ kommen z.B. tBu, Bzl oder Ac in Betracht, wobei Phenylalanin, Alanin, Serin und Valin bevorzugte Aminosäuren sind.

R₂ bedeutet vorzugsweise Bz oder Boc.

Als Salze kommen z.B. Hydrobromide, Hydrochloride, Trifluoracetate oder Acetate in Betracht, wobei Acetat- Trifluoracetat- und Hydrochlorid-Salze bevorzugt sind,

Die oben verwendeten Abkürzungen für Schutzgruppen sowie eine Reihe weiterer Abkürzungen werden in einer zwischen Beispielen 2 und 3 eingefügten Tabelle erläutert.

Die Erfindung betrifft auch die folgenden Verbindungen der in Anspruch 1 definierten Formel I, worin R₁ NHC(NH)NH₂ oder CH₂NH₂ AA₂ Lys(ε-Z) oder lys(ε-Boc) AA₃ Ala, Ser, Phe,Val, Ser(Bzl) oder Ser (Ac) n 0 oder 1 R₂ Bz, Boc oder Z bedeuten

Die Verbindungen der allgemeinen Formel I, welche bezüglich dem mit dem Schwefelhaltigen Substituenten versehenen asymmetrischen Kohlenstoff als Racemate oder in einer ihrer enantiomerenreinen Formen D und L vorliegen können, lassen sich nach den nachfolgend beschriebenen, an sich bekannten Methoden herstellen.

Geschützte Aminosäuren oder geschützte Oligopeptidderivate werden in die entsprechenden Säureamide übergeführt, und diese werden analog zu den von Hong, N.J. et al, Bull. Korean Chem. Soc. 1998, 19(2), 189-193 beschriebenen Verfahren mit einem Alkylester der Glyoxylsäure, zweckmässigerweise einem C₁₋₆-Alkylester, wie Glyoxylsäureethylester, unter Rückfluss kondensiert. Die dabei entstandenen Hydroxyglycinderivate werden O-acetyliert und mit einer entsprechenden Mercaptoalkylverbindung, wie Cysteamin, 3-Mercaptopropylamin oder S-2-Aminoethylisothiouroniumbromid, substituiert. Die erhaltenen Alkylester der Verbindungen der Formel I werden dann zu den entsprechenden freien Säuren alkalisch verseift. Die enantiomerenreinen Säuren können durch eine enantioselektive Hydrolyse der Ester mit Hilfe geeigneter Enzyme erhalten werden.

Die Verbindungen der Formel I und ihre Säureadditionssalze eignen sich als Substrate für die Bestimmung von TAFIa. Diese Bestimmung kann erfindungsgemäss dadurch durchgeführt werden, dass man TAFIa in Gegenwart von 5,5'-Dithiobis-(2-nitrobenzosäure), unter der Bezeichnung "Ellman's Reagens" bekannt, auf eine Verbindung gemäss einem der Ansprüche 1 bis 7 einwirken lässt und die dabei durch die Bildung von 3-Carboxy-4-nitrothiophenol entstehende Absorption zwischen 400 und 412 nm in Abhängigkeit von der Zeit photospektrometisch ermittelt. Die erwähnte Einwirkung erfolgt zweckmässigerweise bei etwa 10°C bis 37°C, vorzugsweise bei Raumtemperatur, und die Einwirkungszeit kann etwa 5 bis 15 Minuten, vorzugsweise etwa 10 Minuten, betragen. Als Quelle für TAFIa kann das im Blutplasma vorhandene TAFI verwendet werden.

Die nachfolgenden Beispiele sollen die Erfindung näher illustrieren ohne ihren Umfang einzuschränken.

Die Identifizierung und Charakterisierung der gemäss Beispielen 1 und 2 erhaltenen Eluate und Produkte wurde mittels Proton-NMR, HPLC-Elektrospray-MS und/oder Elementaranalyse ausgeführt.

### Beispiel 1:

### Herstellung von Benzoyl-(L)-lysyl-(ε-benzyloxycarbonyl)-α-(D,L)-(2-guanidinoethylthio)-glycin Hydrochloridsalz [Bz-(L)-Lys(Z)-(D,L)-Gly (α-GUS)-OH•HCl]

### 1A) Benzoyl(L)-lysyl(ε-benzyloxycarbonyl)-α-(D,L)-hydroxyglycine-ethylester [(Bz-(L)-Lys(Z)-(D,L)-Gly(α-OH)-Oet]

5.0 g (13 mmol) Benzoyl-(L)-lysinamid und eine 50%-ige Toluollösung von 13.3 g (65 mmol) Glyoxylsäureethylester wurden in 100ml THF rückfliessend erhitzt. Nach 4h wurde die Lösung auf RT abgekühlt und direkt in den Reaktionsschritt 1 B) überführt.

### 1B) Benzoyl-(L)-lysyl(ε-benzyloxycarbonyl)-α-(D,L)-acetoxyAlycine-ethylester [Bz-(L)-Lys(Z)-(D,L)-Gly(α-Oac)-Oet]

Die Reaktionslösung aus 1A) wurde mit 127 mg (1 mmol) DMAP, 24.7 ml (0.31 mol) Pyridin und 40 g (0.39 mol) Essigsäureanhydrid versetzt und bei RT während 90 Min. gerührt. Die Reaktionslösung wurde über Kieselgel filtriert, das Lösungsmittel abdestilliert und das erhaltene Rohprodukt im Vakuumtrockenschrank getrocknet. Ausbeute: 14 g.

### 1C) Benzoyl-(L)-lysyl-(ε-benzyloxycarbonyl)-α-(D,L)-(2-guanidinoethylthio)-glycine ethylester [Bz-(L)-Lys(Z)-(D,L)-Gly(α-GUS)-OEt]

5.5 g (19.5 mmol) S-(2-Aminoethyl)isothiouroniumbromid hydrobromid und 4 g (39 mmol) TEA wurden in 50 ml DMF gelöst und während 5 Min. bei RT gerührt. Dann wurde eine Lösung von 14 g des unter 1B) erhaltenen Produktes in 30 ml DMF zugegeben. Nach 2 h Rühren bei RT wurde das Lösungsmittel am Rotovapor abdestilliert. Das erhaltene Rohprodukt wurde mittels LH2O (Methanol) chromatographiert. Die Verbindung fällt als gelbliches Öl an.
ESI-MS [M+H]- 587

### 1D) Benzoyl-(L)-lysyl-(ε-benzyloxycarbonyl)-α-(D,L)-(2-guanidinoethyldinoethylthio)-glycine Hydrochloridsalz [Bz-(L)-Lys(Z)-(D,L)-Gly(α-GUS)-OH • HCl]

3.1 g (4.4 mmol) der Verbindung 1C) wurden in 20 ml Ethanol gelöst, mit 11 ml NaOH (1 N) versetzt und über Nacht bei RT gerührt. Nach Einstellen des pH's auf 3 mit Zitronensäure und Abdestillieren des Lösungsmittels wurde der Rückstand mittels LH2O (Methanol) chromatographiert. Zur Überführung in das Hydrochloridsalz wurden die reinen, eingeengten Fraktionen in 100 ml eines Methanol/Wasser-Gemisches (1:1) gelöst und über Amberlite (Cl⁻ Form) chromatographiert. Die erhaltene Lösung wurde am Rotovapor eingeengt und das Produkt mittels Zugabe von BME als nahezu weisses, amorphes Pulver ausgefällt. Das Pulver wurde abfiltriert, mit wenig BME gewaschen und im Vakuumtrockenschrank getrocknet.
Ausbeute: 2.5 g, ESI-MS [M+H]- 559

### Beispiel 2:

### Herstellung von Benzoyl-(L)-lysyl-(ε-benzyloxycarbonyl)-α-(D,L)-(3-aminopropylthio)-glycine Trifluoressigsäuresalz [Bz-(L)-Lys(Z)-(D,L)-Gly(a-SprA)-OH • TFA]

### 2C) Benzoyl-(L)-lysyl-(ε-benzyloxycarbonyl)-α-(D,L)-(3-aminopropylthio)-glycine ethylester Trifluoressigsäuresatz [Bz-(L)-Lys(Z)-(D,L)-Gly(α-SprA)-Oet • TFA]

2.5 g (20 mmol) 3-Mercaptopropylamine hydrochlorid und 2 g (20 mmol) TEA wurden in 40 ml DMF gelöst, die Lösung während 5 min bei RT gerührt und mit einer Lösung von 6.9 g des unter 1B) erhaltenen Produktes in 30 ml DMF versetzt. Nach 2-stündigem Rühren bei RT wurde das Lösungsmittel am Rotovapor abdestilliert. Das erhaltene Rohprodukt wurde mittels präparativer HPLC
chromatographiert.(ACN/H₂O/TFA). Die reinen Fraktionen wurden am Rotovapor eingeengt.
ESI-MS [M+H] - 559

### 2D) Benzoyl-(L)-lysyl-(ε-benzyloxycarbonyl)-α-(D,L)-(3-aminopropylthio)-glycine Trifluoressigsäuresalz [Bz-(L)-Lys(Z)-(D,L)-Gly(α-SprA)-OH•TFA]

0.43 g (0.6 mmol) der Verbindung 2C) wurden in 7 ml Ethanol gelöst, mit 2 ml NaOH (1 N) versetzt und über Nacht bei RT gerührt. Die Reaktionslösung wurde mit 10%-iger Zitronensäure auf pH 3 gestellt, 3 mal mit je 20 ml Ethylacetat extrahiert und mit ges. NaCl-Lösung 2 mal gewaschen. Die organische Phase wurde über Na₂SO₄ getrocknet, filtriert und das Lösungsmittel am Rotovapor abdestilliert. Das Rohprodukt wurde mittels präparativer HPLC chromatographiert (ACN/H₂O/TFA) Die reinen Fraktionen wurden am Rotovapor eingeengt und der Rückstand in wenig Methanol gelöst und durch Zugabe von BME ausgefällt. Das nahezu weisse Pulver wurde abfiltriert, mit wenig BME gewaschen und im Vakuumtrockenschrank getrocknet.
ESI-MS [M+H] - 531

### Abkürzungen

| | |
|---|---|
| Ac | Acetyl |
| ACN | Acetonitril |
| Aloc | Allyloxycarbonyl |
| BME | t-Butyl methyl ether |
| Boc | t-Butyloxycarbonyl |
| Bz | Benzoyl |
| Bzl | Benzyl |
| DMAP | 4-(Dimethylamino)-pyridin |
| DMF | N,N-Dimethylformamid |
| Dnp | 2,4-Dinitrophenyl |
| DTNB | 5,5'-Dithiobis-(2-nitrobenzosäure) |
| EtOAc | Ethylacetat |
| GUS | 2-Guanidino-ethylthio |
| HEPES | 4-(2-Hydroxyethyl)-piperazin-1-ethansulfonsäure |
| LH20 | Sephadex |
| MeoSuc | Methoxysuccinyl |
| NAPAP | Nα-(2-naphthylsulfonyl-glycyl)-4-amidinophenylalanine-piperidide |
| Pbf | 2,2,4,6,7-Pentamethyl-dibenzohydrofuran-5-sulfonyl |
| SprA | 3-Aminopropylthio |
| RT | Raumtemperatur |
| Suc | Succinyl |
| tBu | tert.-Butyl |
| TEA | Triethylamin |
| TFA | Trifluoressigsäure |
| THF | Tetrahydrofuran |
| Tos | Tosyl |
| Z | Benzyloxycarbonyl |

### Beispiel 3:

### Quantitative Bestimmung von TAFIa

Prinzip: TAFIa wird analog zu Schema 1 bestimmt. In einem ersten Schritt wird das Substrat von TAFIa in der Weise hydrolisiert, dass eine Thioaminosäure (Thiaarginin oder Thialysin) freigesetzt wird. Diese instabilen Zwischenprodukte werden rasch zu 2-Mercaptoethylguanidin resp. 3-Mercaptopropylamin abgebaut. Diese Mercaptoverbindungen reagieren mit dem Ellman's Reagens (5,5'-Dithiobis-(2-nitro benzoesäure)) und setzen dabei das intensive gelbe 3-Carboxy-4-nitrothiophenol frei, welches photospektrometrisch bei 405-412 nm gemessen werden kann. Der gemessene Gehalt an freiem Thiol ist direkt proportional zur Menge des durch TAFIa hydrolisierten Substrats und ermöglicht damit eine quantitative Analyse der EnzymAktivität.

Enzym-Assay: Die Messung der Enzymaktivität wird unter Verwendung einer Substrat Stammlösung von 10 mM in DMSO bei Raumtemperatur durchgeführt. TAFI menschlichen Ursprungs ist in einer Stammlösung von 360µg/ml bei Enzyme Research Laboratory oder über Blutplasma erhältlich. Die Aktivierung von TAFI zu TAFIa wurde mittels Thrombin (2.7U/ml), dessen Aktivierung mit 30 µg Thrombomodulin erfolgte, vorgenommen. Überschüssige Thrombinaktivität wurde durch Zugabe des synthetischen Inhibitors NAPAP eliminiert. Die Messung wurde auf Mikrotiterplatten durchgeführt.

Die Zunahme der Absorption bei 405-412 nm wurde während 10 Minuten aufgezeichnet.

### Aktivierung:

| | |
|---|---|
| TAFI | 20 µl |
| Thrombin (2,7 E/ml) | 20 µl |
| Thrombomodulin (30 µg/ml) | 20 µl |
| in Puffer (20 mM HEPES, 5 mM CaCl2, 0,01 % Tween 80; pH 7,4) 5 min bei 25 °C inkubieren | |

### Messung:

| | |
|---|---|
| Puffer (20 mM HEPES, 5 mM CaCl2, 0,01 % Tween 80; pH 7,4) | 120 µl |
| NAPAP (100 µM) | 20 µl |
| DTNB (5 mM) | 10 µl |
| Substrat (10 mM) | 20 µl |
| | bei RT 10 min messen |

### Resultate:

- Spaltung von Thiaarginin- und Thialysinsubstraten (Endkonzentration 174 µM) durch TAFIa (Endkonzentration 17 µg/ml)
- in Klammern die Werte für Carboxypeptidase B
- Vergleichssubstanzen A und B aus "Bull. Korean.
   Chem. Soc. 1998, 19(2), 189-193"
- ΔE = Änderung der Extinktion bei 405 nm

**Tabelle 1**

| **Nr.** | **Substrate** | **ΔE/min** | |
|---|---|---|---|
| 4269 | Bz-K(Z)-G(GUS)-OH X HCl | 0,108 | (0,018) |
| 4273 | Boc-K(Tos)-G(GUS)-OH x HCl | 0,014 | (0,048) |
| 4275 | Boc-K(Z)-G(GUS)-OH x TFA | 0,041 | (0,028) |
| 4298 | Boc-F-K(Boc)-G(GUS)-OH x HCl | 0,043 | (0,034) |
| 4300 | Bz-P-K(Boc)-G(GUS)-OH x TFA | 0,012 | (0,072) |
| 4302 | Z-A-K(Boc)-G(GUS)-OH x HCl | 0,042 | (0,059) |
| 4334 | Bz-V-G(GUS)-OH x TFA | 0,034 | |
| 4336 | Bz-V-G(α-SPrA)-OH x TFA | 0,053 | |
| 4339 | Bz-K(Z)-G(α-SPrA)-OH x TFA | 0,055 | |
| 4341 | Boc-K(Z)-G(α-SPrA)-OH x TFA | 0,054 | |
| | Vergleichssubstanz A x HCl | 0,002 | (0,062) |
| | Vergleichssubstanz B x HCl | 0,016 | (0,077) |

## Patentansprüche

1. Verwendung von Verbindungen der allgemeinen Formel I 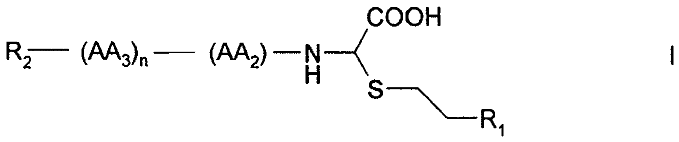 worin
R₁ eine Gruppe CH₂NH₂ oder NHC(NH)NH₂ bedeutet,
AA₂ jeweils unsubstituiertes oder substituiertes Lysin, Ornithin, Arginin oder Histidin bedeutet, wobei die Substituenten übliche Schutzgruppen sind,
AA₃ eine natürliche Aminosäure bedeutet, bei der eine allenfalls vorhandene schützbare Gruppe in der Seitenkette durch eine übliche Schutzgruppe substituiert sein kann,
n 0 oder 1 ist, und
R₂ eine Gruppe Bz, Bzl, Ac, Boc, Z, Suc, MeoSuc oder Tos bedeutet,
mit der Massgabe, dass nicht gleichzeitig n 0 ist und R₂ Z und (AA₂) unsubstituiertes oder durch Boc substituiertes Lysin bedeuten,
als Racemate oder als enantiomerenreine Isomere,
und von deren Salzen mit Mineralsäuren oder organischen Säuren, als Substrate für die Bestimmung von TAFIa.

2. Verwendung gemäss Anspruch 1, wobei AA₂ Lys(ε-Z), Lys(ε-Boc), Lys(ε-Ac), Lys(ε-Bz), Lys(ε-Bzl), Lys(ε-Tos), Orn(δ-Z), Orn(δ-Boc), Orn(δ-2-chlor-Z), Orn(δ-Dnp), Orn(δ-Z), Orn(δ-Aloc), Arg(ω-Pbf), Arg(δ,ω-Boc)₂, Arg(δ,ω-Z)₂, Arg(ω-Tos), His(N^{im}-Boc), His(N^{im}-Ac), His(N^{im}-Bz),His(N^{im}-Bzl) oder His(N^{im}-Tos) bedeutet, wobei Lys(ε-Z) bevorzugt ist

3. Verwendung gemäss Anspruch 1 oder 2, wobei AA₃ Ala, Ser, Phe, Val, Ile, Leu, Thr, Pro, Lys, Arg, His, Asp, Glu, Asn, Gln, Cys, Met, Trp, Tyr oder Gly bedeuten, wobei eine allenfalls vorhandene schützbare Gruppe in der Seitenkette durch eine übliche Schutzgruppe, wie tBu, Bzl oder Ac substituiert sein kann.

4. Verwendung gemäss Anspruch 3, wobei AA₃ Phe, Ala, Val oder gegebenenfalls durch tBu, Bzl oder Ac geschütztes Ser bedeutet.

5. Verwendung gemäss Anspruch 1, wobei
R₁ NHC(NH)NH₂,
AA₂ Lys(ε-Z) oder Lys(ε-Boc),
AA₃ Ala, Ser, Phe, Val, Ser(tBu), Ser(Bzl) oder Ser(Ac),
n 0 oder 1 und
R₂ Bz, Boc oder Z bedeuten.

6. Verwendung gemäss Anspruch 1, wobei
R₁ CH₂NH₂,
AA₂ Lys(ε-Z) oder Lys(ε-Boc),
AA₃ Ala, Ser, Phe, Val, Ser(tBu), Ser(Bzl) oder Ser(Ac),
n 0 oder 1 und
R₂ Bz, Boc oder Z bedeuten.

7. Verwendung gemäss einem der Ansprüche 1 bis 6, **dadurch gekennzeichnet, dass** die Verbindungen als Säureadditionssalze in Form von Hydrobromiden, Hydrochloriden, Trifluoracetaten oder Acetaten vorliegen.

8. Verbindungen der in Anspruch 1 definierten Formel I, worin
R₁ NHC(NH)NH₂,
AA₂ Lys(ε-Z) oder Lys(ε-Boc),
AA₃ Ala, Ser, Phe, Val, Ser(tBu), Ser(Bzl) oder Ser(Ac),
n 0 oder 1 und
R₂ Bz, Boc oder Z bedeuten.

9. Verbindungen der in Anspruch 1 definierten Formel I, worin
R₁ CH₂NH₂,
AA₂ Lys(ε-Z) oder Lys(ε-Boc),
AA₃ Ala, Ser, Phe, Val, Ser(tBu), Ser(Bzl) oder Ser(Ac),
n 0 oder 1 und
R₂ Bz, Boc oder Z bedeuten.

10. Verbindung gemäss Anspruch 8, worin AA₂ Lys(ε-Z), n 0 und R₂ Bz bedeuten.

11. Verbindungen gemäss einem der Ansprüche 8 bis 10, **dadurch gekennzeichnet, dass** diese als Säureadditionssalze in Form von Hydrobromiden, Hydrochloriden, Trifluoracetaten oder Acetaten vorliegen.

12. Verwendung der Verbindungen gemäss einem der Ansprüche 8 bis 11 als Substrate für die Bestimmung von TAFIa.

13. Verfahren zur Bestimmung von TAFIa, **dadurch gekennzeichnet, dass** man TAFIa in Gegenwart von 5,5'-Dithiobis-(2-nitrobenzosäure) auf eine Verbindung der in Anspruch 1 definierten Formel I bzw. gemäss einem der Ansprüche 8 bis 11 einwirken lässt und die dabei durch die Bildung von 3-Carboxy-4-nitrothiophenol entstehende Absorption zwischen 400 und 412 nm in Abhängigkeit von der Zeit photospektrometisch ermittelt.

14. Verfahren gemäss Anspruch 13, **dadurch gekennzeichnet, dass** die Einwirkung während 5 bis 15 Minuten, insbesondere während 10 Minuten bei einer Temperatur von 10°C bis 37°C, insbesondere bei Raumtemperatur erfolgt.

15. Verfahren gemäss Anspruch 13 oder 14 **dadurch gekennzeichnet, dass** als Quelle für TAFIa das im Blutplasma vorhandene TAFI verwendet wird.

16. Verfahren gemäss Anspruch 15 zur Bestimmung von TAFI im Blutplasma, **dadurch gekennzeichnet, dass** man dieses TAFI mittels Thrombomodulin und daran gebundenem Thrombin zu TAFIa aktiviert, überschüssige Thrombinaktivität durch Zugabe von NAPAP eliminiert, das entstandene TAFIa in Gegenwart von 5,5'-Dithiobis-(2-nitrobenzoesäure) auf eine Verbindung der in Anspruch 1 definierten Formel I bzw. gemäss einem der Ansprüche 8 bis 11 einwirken lässt und die dabei durch die Bildung von 3-Carboxy-4-nitrothiophenol entstehende Absorption zwischen 400 und 412 nm in Abhängigkeit von der Zeit photospektrometrisch ermittelt.

17. Verfahren gemäss Anspruch 16, **dadurch gekennzeichnet, dass** die Menge an TAFI 20 Volumenteilen einer Stammlösung von 360 µg/ml, die Menge an Thrombomodulin 20 Volumenteilen einer Lösung von 30 µg/ml, die Menge an Thrombin 20 Volumenteilen einer Lösung von 2.7 E/ml, die Menge an NAPAP 20 Volumenteilen einer Lösung von 100 µM/ml, die Menge an 5,5'-Dithiobis-(2-nitrobenzoesäure) 10 Volumenteilen einer Lösung von 5 mM/ml und die Menge einer Verbindung der in Anspruch 1 definierten Formel I bzw. gemäss einem der Ansprüche 8 bis 11 20 Volumenteilen einer Lösung von 10 mM/ml entsprechen.

18. Verfahren zur Herstellung der Verbindungen gemäss einem der Ansprüche 8 bis 10, **dadurch gekennzeichnet, dass** man einen entsprechenden Alkylester alkalisch verseift.

## Claims

1. Use of compounds having the general Formula I 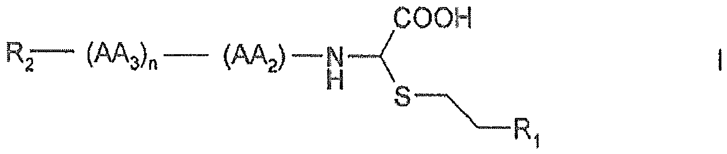 wherein
R₁ stands for a group CH₂NH₂ or NHC(NH)NH₂,
AA₂ stands for unsubstituted or substituted lysine, orthinine, arginine, or histidine, wherein the substituents are usual protective groups,
AA₃ stands for a natural amino acid, in which a protectable group in the side chain, which might be present, can be substituted by a usual protective group,
n is 0 or 1, and
R₂ stands for a group Bz, Bzl, Ac, Boc, Z, Suc, MeoSuc, or Tos,
with the proviso that it cannot hold true at the same time that n is 0 and R₂ stands for Z and (AA₂) stands for unsubstituted lysine or lysine substituted by Boc,
as a racemate or as enantiomer-pure isomers,
and of their salts with mineral acids or organic acids, as substrates for the determination of TAFIa.

2. Use according to claim 1, wherein AA₂ stands for Lys(ε-Z), Lys(ε-Boc), Lys(ε-Ac), Lys(ε-Bz), Lys(ε-Bzl), Lys(ε-Tos), Orn(δ-Z), Orn(δ-Boc), Orn(δ-2-chloro-Z), Orn(δ-Dnp), Orn(δ-Z), Orn(δ-Aloc), Arg(ω-Pbf), Arg(δ,ω,-Boc)₂, Arg(δ,ω-Z)₂, Arg(ω-Tos), His(N^{im}-Boc), His(N^{im}-Ac), His(N^{im}-Bz), His(N^{im}-Bzl), or His(N^{im}-Tos), wherein Lys(ε-Z) is preferred.

3. Use according to claim 1 or 2, wherein AA₃ stand for [sic - plural verb] Ala, Ser, Phe, Val, Ile, Leu, Thr, Pro, Lys, Arg, His, Asp, Glu, Asn, Gln, Cys, Met, Trp, Tyr, or Gly, wherein a protectable group in the side chain, which might be present, can be substituted by a usual protective group, such as tBu, Bzl, or Ac.

4. Use according to claim 3, wherein AA₃ stands for Phe, Ala, Val, or, if applicable, for Ser protected by tBu, Bzl, or Ac.

5. Use according to claim 1, wherein
R₁ stands for NHC(NH)NH₂,
AA₂ stands for Lys(ε-Z), or Lys(ε-Boc),
AA₃ stands for Ala, Ser, Phe, Val, Ser(tBu), Ser(Bzl), or Ser(Ac),
n stands for 0 or 1, and
R₂ stands for Bz, Boc, or Z.

6. Use according to claim 1, wherein
R₁ stands for CH₂NH₂,
AA₂ stands for Lys(ε-Z), or Lys(ε-Boc),
AA₃ stands for Ala, Ser, Phe, Val, Ser(tBu), Ser(Bzl), or Ser(Ac),
n stands for 0 or 1, and
R₂ stands for Bz, Boc, or Z.

7. Use according to one of claims 1 to 6, **characterized in that** the compounds are present as acid addition salts in the form of hydrobromides, hydrochlorides, trifluoroacetates, or acetates.

8. Compounds having the Formula I defined in claim 1, wherein
R₁ stands for NHC(NH)NH₂,
AA₂ stands for Lys(ε-Z), or Lys(ε-Boc),
AA₃ stands for Ala, Ser, Phe, Val, Ser(tBu), Ser(Bzl), or Ser(Ac),
n stands for 0 or 1, and
R₂ stands for Bz, Boc, or Z.

9. Compounds having the Formula I defined in claim 1, wherein
R₁ stands for CH₂NH₂,
AA₂ stands for Lys(ε-Z), or Lys(ε-Boc),
AA₃ stands for Ala, Ser, Phe, Val, Ser(tBu), Ser(Bzl), or Ser(Ac),
n stands for 0 or 1, and
R₂ stands for Bz, Boc, or Z.

10. Compounds according to claim 8, wherein AA2 stands for Lys(ε-Z), n stands for 0, and R₂ stands for Bz.

11. Compounds according to one of claims 8 to 10, **characterized in that** these are present as acid addition salts in the form of hydrobromides, hydrochlorides, trifluoroacetates, or acetates.

12. Use of the compounds according to one of claims 8 to 11 as substrates for the determination of TAFIa.

13. Method for the determination of TAFla, **characterized in that** TAFIa is allowed to act, in the presence of 5,5'-dithiobis-(2-nitrobenzoic acid), on a compound having the Formula I defined in claim 1, or according to one of claims 8 to 11, and in this connection, the absorption between 400 and 412 nm that occurs due to the formation of 3-carboxy-4-nitrothiophenol is determined by photospectrometry, as a function of time.

14. Method according to claim 13, **characterized in that** the action takes place during 5 to 15 minutes, particularly during 10 minutes, at a temperature of 10°C to 37°C, particularly at room temperature.

15. Method according to claim 13 or 14, **characterized in that** the TAFI present in the blood plasma is used as a source for TAFIa.

16. Method according to claim 15, for determination of TAFI in blood plasma, **characterized in that** this TAFI is activated, by means of thrombomodulin and thrombin bound to it, to produce TAFIa, excess thrombin activity is eliminated by means of addition of NAPAP, the resulting TAFIa is allowed to act, in the presence of 5,5'-dithiobis-(2-nitrobenzoic acid), on a compound having the Formula I defined in claim 1, or according to one of claims 8 to 11, and in this connection, the absorption between 400 and 412 nm that occurs due to the formation of 3-carboxy-4-nitrothiophenol is determined by photospectrometry, as a function of time.

17. Method according to claim 16, **characterized in that** the amount of TAFI corresponds to 20 parts by volume of a master solution of 360 µg/ml, the amount of thrombomodulin amounts to 20 parts by volume of a solution of 30 µg/ml, the amount of thrombin amounts to 20 parts by volume of a solution of 2.7 U/ml, the amount of NAPAP amounts to 20 parts by volume of a solution of 100 µM/ml, the amount of 5,5'-dithiobis-(2-nitrobenzoic acid) amounts to 10 parts by volume of a solution of 5 mM/ml, and the amount of a compound having the Formula I defined in claim 1 or according to one of claims 8 to 11 amounts to 20 parts by volume of a solution of 10 mM/ml.

18. Method for the production of the compounds according to one of claims 8 to 10, **characterized in that** a corresponding alkyl ester is saponified in alkaline manner.

## Revendications

1. Utilisation de composés de la Formule générale I : 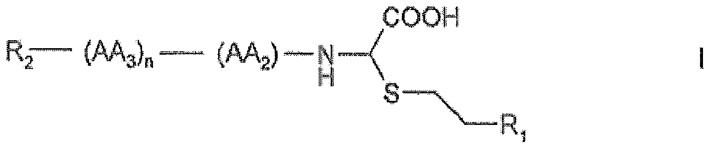 dans laquelle :
- R₁ signifie un groupe CH₂NH₂ ou NHC(NH)NH₂ ;
- AA₂ signifie lysine, ornithine, arginine ou histidine respectivement non substituée ou substituée, les substituants étant des groupes protecteurs usuels ;
- AA₃ représente un acide aminé naturel, pour lequel un groupe protégeable éventuellement présent dans la chaîne latérale peut être substitué par un groupe protecteur usuel ;
- n vaut 0 ou 1 ; et
- R₂ signifie un groupe Bz, Bzl, Ac, Boc, Z, Suc, MeoSuc ou Tos,
à la condition que l'on n'ait pas simultanément n égal à 0 et R₂ représentant Z et (AA₂) représentant lysine non substituée ou substituée par Boc,
en tant que racémates ou en tant qu'isomères énantiomériquement purs,
et de leurs sels avec des acides minéraux ou acides organiques, comme substrats pour la détermination de TAF1a.

2. Utilisation selon la revendication 1, suivant laquelle AA₂ signifie Lys(ε-Z), Lys(ε-Boc), Lys(ε-Ac), Lys(ε-Bz), Lys(ε-Bzl), Lys(ε-Tos), Orn(δ-Z), Orn(δ-Boc), Orn(δ-2-chlor-Z), Orn(δ-Dnp), Orn(δ-Z), Orn(δ-Aloc), Arg(ω-Pbf), Arg(δ,ω-Boc)₂, Arg(δ,ω-Z)₂, Arg((ω-Tos), His(N^{im}-Boc), His(N^{im}-Ac), His(N^{im}-Bz), His(N^{im}-Bzl) ou His(N^{im}-Tos), Lys(ε-Z) étant préféré.

3. Utilisation selon l'une des revendications 1 ou 2, suivant laquelle AA₃ signifie Ala, Ser, Phe, Val, Ile, Leu, Thr, Pro, Lys, Arg, His, Asp, Glu, Asn, Gln, Cys, Met, Trp, Tyr ou Gly, un groupe protégeable éventuellement présent dans la chaîne latérale pouvant être substitué dans la chaîne latérale par un groupe protecteur usuel tel que tBu, Bzl ou Ac.

4. Utilisation selon la revendication 3, dans laquelle AA₃ signifie Phe, Ala, Val ou Ser éventuellement protégé par tBu, Bzl ou Ac.

5. Utilisation selon la revendication 1, selon laquelle
- R₁ signifie NHC(NH)NH₂ ;
- AA₂ signifie Lys(ε-Z) ou Lys(ε-Boc) ;
- AA₃ signifie Ala, Ser, Phe, Val, Ser(tBu), Ser(Bzl) ou Ser(Ac) ;
- n vaut 0 ou 1 ; et
- R₂ signifie Bz, Boc ou Z.

6. Utilisation selon la revendication 1, suivant laquelle
- R₁ signifie CH₂NH₂ ;
- AA₂ signifie Lys(ε-Z) ou Lys(ε-Boc) ;
- AA₃ signifie Ala, Ser, Phe, Val, Ser(tBu), Ser(Bzl) ou Ser(Ac) ;
- n vaut 0 ou 1 ; et
- R₂ signifie Bz, Boc ou Z.

7. Utilisation selon l'une des revendications 1 à 6, **caractérisée par le fait que** les composés en tant que sels d'addition avec les acides se présentent sous la forme de bromhydrates, chlorhydrates, trifluoracétates ou acétates.

8. Composés de la Formule I définie dans la revendication 1, dans desquels
- R₁ signifie NHC (NH) NH_{z} ;
- AA₂ signifie Lys(ε-Z) ou Lys(ε-Boc) ;
- AA₃ signifie Ala, Ser, Phe, Val, Ser(tBu), Ser(Bzl) ou Ser(Ac)
- n vaut 0 ou 1 ; et
- R₂ signifie Bz, Boc ou Z.

9. Composés de la Formule I définie dans la revendication 1, dans desquels
- R₁ signifie CH₂NH₂ ;
- AA₂ signifie Lys(ε-Z) ou Lys(ε-Boc) ;
- AA₃ signifie Ala, Ser, Phe, Val, Ser(tBu), Ser (Bzl) ou Ser(Ac) ;
- n vaut 0 ou 1 ; et
- R₂ signifie Bz, Boc ou Z.

10. Composé selon la revendication 8, dans lequel AA₂ signifie Lys(ε-Z), n vaut 0 et R₂ signifie Bz.

11. Composés selon l'une des revendications 8 à 10, **caractérisés par le fait qu'**en tant que sels d'addition avec les acides, ceux-ci se présentent sous la forme de bromhydrates, chlorhydrates, trifluoracétates ou acétates.

12. Utilisation des composés selon l'une des revendications 8 à 11 comme substrats pour la détermination de TAF1a.

13. Procédé de détermination de TAF1a, **caractérisé par le fait que** l'on laisse agir TAF1a en présence d'acide 5,5-dithiobis-(2-nitrobenzoïque) sur un composé de la Formule I définie dans la revendication 1 ou selon l'une des revendications 8 à 11, et que l'on détermine par photospectrométrie l'absorption occasionnée par la formation de 3-carboxy-4-nitrothiophénol entre 400 et 412 nm en fonction du temps.

14. Procédé selon la revendication 13, **caractérisé par le fait que** l'action a lieu pendant 5 à 15 minutes, en particulier pendant 10 minutes à une température de 10°C à 37°C, en particulier à la température ambiante.

15. Procédé selon l'une des revendications 13 ou 14, **caractérisé par le fait que** l'on utilise comme source pour TAF1a le TAF1 présent dans le plasma sanguin.

16. Procédé selon la revendication 15 pour la détermination de TAF1 dans le plasma sanguin, **caractérisé par le fait que** l'on active ce TAF1 au moyen de la thrombomoduline et de la thrombine liée à celle-ci en TAF1a, on élimine l'activité thrombine en excès par addition de NAPAP, on laisse agir le TAF1a qui se forme en présence d'acide 5,5'-dithiobis-(2-nitrobenzoïque) sur un composé de la Formule I définie dans la revendication 1 ou selon l'une des revendications 8 à 11, et on détermine par photospectrométrie l'absorption occasionnée lors de cela par la formation de 3-carboxy-4-nitrophénol entre 400 et 412 nm en fonction du temps.

17. Procédé selon la revendication 16, **caractérisé par le fait que** la quantité de TAF1 correspond à 20 parties en volume d'une solution-mère de 360 µg/ml, la quantité de thrombomoduline correspond à 20 parties en volume d'une solution de 30 µg/ml, la quantité de thrombine correspond à 20 parties en volume d'une solution de 2,7 E/ml, la quantité de NAPAP correspond à 20 parties en volume d'une solution de 100 µM/ml, la quantité d'acide 5,5'-dithiobis-(2-nitrobenzoïque) correspond à 10 parties en volume d'une solution de 5 mM/ml, et la quantité d'un composé de la Formule I définie à la revendication 1 ou selon l'une des revendications 8 à 11 correspond à 20 parties en volume d'une solution de 10 mM/ml.

18. Procédé de fabrication des composés selon l'une des revendications 8 à 10, **caractérisé par le fait que** l'on conduit une saponification alcaline d'un ester alkylique correspondant.
